# EUROPEAN PATENT APPLICATION

(11) **EP 2 371 401 A2**
(43) Date of publication of application: **05.10.2011**
(21) Application number: 11001896.7
(22) Date of filing: 08.03.2011
(51) Int. Cl.: A61L 27/20

(54) **A method of production of a cartilage-like biomaterial designed for reconstructive surgery**

(30) Priority: 08.03.2010 PL 39065010
(71) Applicant: Politechnika Lodzka, 90-924 Lodz (PL)
(72) Inventor: Bielecki, Stanislaw, 91-046 Lódz (PL); Kolodziejczyk, Marek, 92-511 Lódz (PL); Kowalska, Karolina, 93-165 Lódz (PL); Krystynowicz, Alina, 91-110 Lódz (PL); Pankiewicz, Teresa, 93-347 Lódz (PL)
(74) Representative: Kaczur-Kaczynska, Ewa

(57) **Abstract**

A method of production of a cartilage-like biomaterial designed for implants for reconstructive surgery by using microbial cellulose produced in a stationary culture of bacterium *Gluconacetobacter xylinus* consists in cultivation of the bacterium in a flat bioreactor or polyethylene tubes, modeling of produced and purified cellulose into spatial construction with desired shape and modification of the latter by treatment with 30% aqueous solution of sodium hydroxide, rinsing with distilled water, treatment with 10% aqueous solution of acetic acid and repeated rinsing with distilled water until pH of cellulose material reaches 5.6-6.8.

## Description

The subject matter of the present invention is a method of fabrication of a cartilage-like biomaterial designed for production of implants intended for reconstructive surgery, such as nasal septum, auricular concha, artificial breast after mastectomy, cranial and tracheal fragments, and protective tubes in end-to-end nerve repair.

Using of biotechnologically produced biomaterials as internal implants allows avoiding problems related to application of biological tissue material such as difficulties in acquisition of the latter material, its conservation, storage, maintenance of microbial purity, resorption, and immune response of host organism to the implanted tissue. Biotechnologically produced biomaterials that are used in reconstructive surgery have to be characterized by both high biocompatibility and appropriate mechanical parameters.

Patents US2007184550, EP1779875, US2005159820 and WO03042376 disclose already known methods of artificial cartilage cultivation by using stem cells, chondrocytes on matrices made from synthetic or natural polymers or hydroxyapatites.

Patents PCT/US2005/036724 and WO03020191 disclose the usage of microbial cellulose as a scaffold in cell cultures while among others from the journal Biomaterials 26, 419-431 (2005*)* it is known cultivation of chondrocytes on scaffolds made from bacterial cellulose in fabrication of an artificial cartilage.

Patent EP0365108 discloses the process of manufacturing of a biocompatible material with mechanical resistance of cartilages made of synthetic polymers and its application as an internal implant.

Patents WO2005003366 and EP1660670 disclose the method of microbial cellulose production consisting in a stationary culture of bacterial strain *Acetobacter xylinum* P₃₀ in culture medium containing glucose, yeast extract, peptone, MgSO₄×7H₂O, Na₂HPO₄, citric acid, ethanol and distilled water, followed by separation of resulting cellulose membrane from culture broth and its purification by rinsing with water, boiling in aqueous NaOH solution, repeated rinsing with water to remove NaOH, treatment with acetic acid, rinsing with tap water and ultimately with distilled water. These patents disclose also the use of resulting cellulose in medicine.

In the present invention, a method of fabrication of a cartilage-like biomaterial designed for production of implants intended for reconstructive surgery, by using of microbial cellulose synthesized in the stationary culture of bacterium *Gluconacetobacter xylinus* in a production medium containing glucose, yeast extract, peptone, MgSO₄x7H₂O, Na₂HPO₄, citric acid, ethanol and distilled water, and purified by rinsing with hot tap water, boiling in 1% aqueous solution of sodium hydroxide or treatment with 1-2% aqueous solution of sodium hydroxide at room temperature, rinsing with tap water, treatment with 1% aqueous solution of acetic acid and repeated rinsing with tap water and ultimately with distilled water, consists in cellulose fabrication through cultivation of bacterium in a flat bioreactor or inside polyethylene tubes, followed by modeling of fabricated and purified cellulose into spatial construction with desired shape which is further subjected to modification consisting in treatment with 30% aqueous solution of sodium hydroxide for 24 hours at room temperature by using 1,5 cm³ of base solution per 1 cm³ of cellulose, rinsing with distilled water, followed by treatment with 10% aqueous solution of acetic acid for at least 2 hours and repeated rinsing with distilled water until pH of the cellulose material reaches 5.6-6.8.

The biomaterial fabricated according to the present invention is characterized by high resistance to tear and rupture, is resilient, can be easily modeled to any shape and retains this shape after modification, is biocompatible and non-allergenic, is characterized by minimized absorption of body fluids and does not undergo resorption under the influence of these fluids. It may serve as a carrier of chemical compounds acting as bacteriostatic, antibacterial and angiogenic agents or growth factors.

The present invention is illustrated by the examples below.

### Example I

An inocular medium with composition given in weight parts: 20 parts of glucose, 5 parts of yeast extract, 5 parts of peptone, 2.5 parts of MgSO₄×7H₂O, 2.7 parts of Na₂HPO₄, 1.15 parts of citric acid, 10 parts of ethanol, filled up to 1000 parts with distilled water was inoculated with 5% (v/v) of suspension of bacterium *Gluconacetobacter xylinus* (5×10⁷ cfu/ml), which was stored on this medium for 7 days at 4°C, and the inoculum was cultivated for 2 days at the temperature of 30°C. The vigorously mixed bacterial suspension was used to inoculate the production medium with the same composition by adding 5% (v/v) of this suspension. The whole inoculated production medium was pre-incubated for 24 hours at the temperature of 30°C and then it was transferred in portions into bioreactors with the surface/volume (SN) ratio of 0.7 cm⁻¹. The culture was carried out in stationary conditions for 7 days and resulting membranes with the thickness of 7-10 mm were purified. For this purpose they were subjected to squeezing of culture broth until achieving 60% of their initial mass, followed by rinsing with hot tap water, treatment with 1 % aqueous solution of sodium hydroxide (NaOH) at 100°C for 1 hour, repeated rinsing with tap water, squeezing up to 40-50% of initial mass, treatment with 1% aqueous solution of acetic acid (CH₃COOH) for 24 hours, repeated rinsing with tap water and ultimately with distilled water. The resulting purified membranes were white with a high degree of transparency and had neutral pH. The excess of water was removed by squeezing until the thickness of membranes was 1.0-4.0 ± 0.03 mm.

The obtained membranes were modified by immersing for 24 hours in 30% aqueous solution of NaOH which was applied in such a volume that 1.5 cm³ of NaOH solution was used per 1 cm³ cellulose. Then the membranes were rinsed with distilled water for 24 hours, immersed in 10% aqueous solution of CH₃COOH for 2 hours and rinsed with distilled water for 24 hours until their pH was 6.6-6.8. The parameters of membranes before and after modification are shown in Table I.

### Example II

An inocular medium with composition given in weight parts: 20 parts of glucose, 5 parts of yeast extract, 5 parts of peptone, 2.5 parts of MgSO₄×7H₂O, 2.7 parts of Na₂HPO₄, 1.15 parts of citric acid, 10 parts of ethanol, filled up to 1000 parts with distilled water was inoculated with 5% (v/v) of suspension of bacterium *Gluconacetobacter xylinus* (5×10⁷ cfu/ml), which was stored on this medium for 7 days at 4°C, and the inoculum was cultivated for 2 days at the temperature of 30°C. The vigorously mixed bacterial suspension was used to inoculate the production medium with the same composition by adding 5% (v/v) of this suspension. The whole inoculated production medium was pre-incubated for 24 hours at the temperature of 30°C then it was transferred to tightly closed polyethylene tubes with internal diameter of 3-10 mm and the culture was carried out for 7 days in stationary conditions. The formed bacterial cellulose tubes with the wall thickness up to 1 mm were purified. For this purpose they were immersed for 24 hours in 1 % aqueous solution of NaOH at room temperature, rinsed with tap water, treated with 1 % aqueous solution of CH₃COOH for 20-24 hours, again rinsed with tap water and ultimately with distilled water. The purified tubes were white, with a high degree of transparency and had neutral pH.

The fabricated tubes were modified as it was described in example I.

Samples of membranes with dimensions of 90x50x3 mm, which were fabricated as described in examples I and II, were tested for tear strength by using a tensile testing machine INSTRON 1112 and the following parameters for all the samples:
- distance between the clamps 50.0 mm
- velocity of cross bar movement 100.0 mm/min
- velocity of recorder tape movement 200.0 mm/min
- measurement scale 20 kg, 50 kg
- temperature 20°C.

Results of tear strength measurements are displayed in Table II.

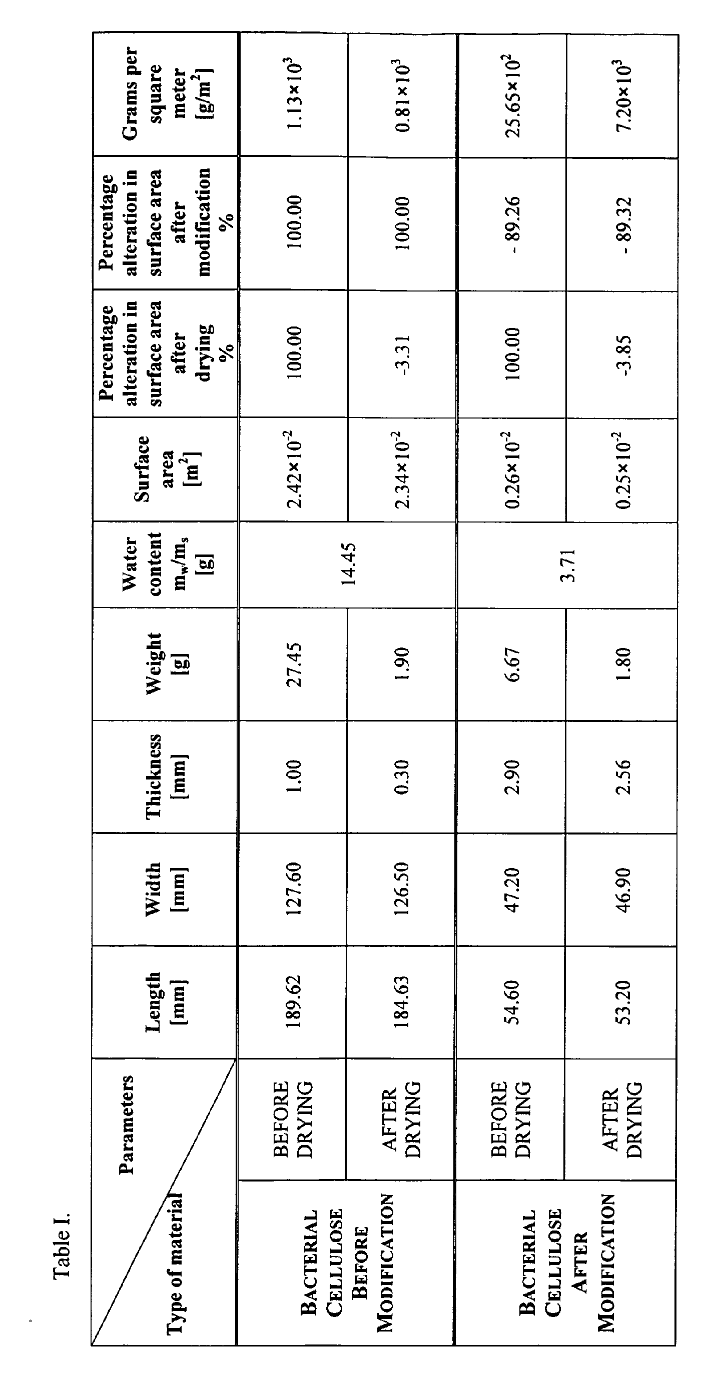

**Table II.**

| **Sample type** | **Thickness [mm]** | **Length [mm]** | **Tear force F [N]** | **Breaking strength R [MPa]** | **Elongation at break %** | **Young modulus E [MPa**] |
|---|---|---|---|---|---|---|
| **UNMODIFIED MICROBIAL CELLULOSE** | 3.0 | 50 | 191.30 | 1.582 | 29 | 2.74 |
| **MODIFIED MICROBIAL CELLULOSE** | 3.0 | 50 | 289.78 | 1.849 | 30 | 1.76 |

The tensile testing machine INSTRON 1112 was also used in measurements of resistance to rupture of the samples of membranes with dimensions of 40×40×3 mm, which were fabricated as described in examples I and II. The following parameters were maintained for all the samples:
- external diameter of the ring used in rupturing force measurement 68.7 mm
- inner diameter of the ring 20.0 mm
- diameter of a metal ball causing the rupture 18.0 mm
- velocity of cross bar movement 100.0 mm/min
- velocity of recorder tape movement 200.0 mm/min
- measurement scale 20 kg, 50 kg
- temperature 20°C.

Results of resistance measurements are shown in Table III.

**Table III.**

| **Type of sample** | **Thickness [mm]** | **Length [mm]** | **Rupturing force F[N]** | **Breaking strength R [MPa]** | **Elongation at break %** | **Young Modulus E [MPa]** |
|---|---|---|---|---|---|---|
| **UNMODIFIED MICROBIAL CELLULOSE** | 3.0 | 40 | 129.49 | 1.496 | 26 | 3.43 |
| **MODIFIED MICROBIAL CELLULOSE** | 3.0 | 40 | 313.63 | 1.960 | 32 | 4.27 |

### Example III

Bacterial cellulose tubes with diameter of 3-10 mm were fabricated and purified as described in example II. Glass rods with diameter of 0.8-1.0 mm were put inside the tubes and the strung material was modified as described in example I.

Resulting tubes were successfully used to connect ends of broken nerve fibers.

### Example IV

Bacterial cellulose membranes, which were fabricated and purified as described in example I, were cut into bars with length of 20-50 mm and cross-section of 5×5 mm, and with length of 30-50 mm and cross-section of 30×30 mm. These bars were pierced with stainless steel needles with diameter of 0.5-2.0 mm or holes with diameter of 2-20 mm were drilled along the axis of cellulose bar. Resulting material was modified as described in example I. Resilient tubes with diameter fitting to the diameter of broken nerve fiber were obtained by this method.

### Example V

Biological *in vivo* tests were carried out for bacterial cellulose fabricated as described in example I, unmodified, modified, and modified and additionally soaked with angiogenic agent.

The *in vivo* tests were performed by using adult male specimens of Wistar rats with weight of approximately 200 g. Before implantation the animals were anaesthetized (according to the protocole) with ketamine and xylazine delivered intraperitoneally in doses of 50 mg/kg and 10 mg/kg, respectively. Rats were put on a surgery table and the skin on their spines was shaved. Then it was disinfected and a cut of approximately 13 mm was made. After separation of skin from the subcutaneous tissue a small pocket was formed into which a sample of bacterial cellulose was put.

The rats were divided into 3 groups with 10 specimens in each group (one control group and 2 test groups).

In the control group, samples of unmodified bacterial cellulose with dimensions of 10 × 10 × 1 mm were implanted into small pockets formed under the skin at the spine of each animal.

In the first group, samples of modified bacterial cellulose were implanted analogously while in the second group samples of cellulose which was modified and soaked with the angiogenic agent were implanted.

Results of cellulose impact on the tissue were evaluated through macroscopic observations performed after 14 and 60 days during explantation of tested samples from the pockets, taking into consideration the level of cellulose integration with host tissue, the density of newly formed blood vessels on cellulose surface and the appearance of the interior of pockets into which cellulose samples were implanted. Explanted samples were subjected to histopathological analysis after staining with hematoxylin and eosin. Performed tests revealed the complete biocompatibility of cellulose, the lack of allergic reactions, rapid covering of cellulose surface with a tissue and blood vessels. Noteworthy, cellulose samples soaked with angiogenic factor were covered by a denser network of blood vessels than the other cellulose samples. Neither swelling nor deformation of implants made of modified bacterial cellulose was observed.

## Claims

1. method of fabrication of a cartilage-like biomaterial designed for implants for reconstructive surgery by using microbial cellulose produced in a stationary culture of bacterium *Gluconacetobacter xylinus,* in a production medium containing glucose, yeast extract, peptone, MgSO₄×7H₂O, Na₂HPO₄, citric acid, ethanol and distilled water, and purified by either rinsing with hot tap water and boiling in 1 % aqueous solution of sodium hydroxide or treatment with 1-2% aqueous solution of sodium hydroxide at room temperature, followed by rinsing with tap water, treatment with 1% aqueous solution of acetic acid and repeated rinsing with tap water and ultimately distilled water, **characterized in that** cellulose is produced through the cultivation of the bacterium in a flat bioreactor or inside polyethylene tubes, and then the produced and purified cellulose is modeled into spatial construction with desired shape and subjected to modification consisting in treatment with 30% aqueous solution of sodium hydroxide for 24 hours at room temperature by using 1.5 cm³ of base solution per 1 cm³ cellulose, rinsing with distilled water, treatment with 10% aqueous solution of acetic acid for at least 2 hours and repeated rinsing with distilled water until pH of cellulose reaches 5.6-6.8.
